# EUROPEAN PATENT APPLICATION

(11) **EP 3 467 126 A1**
(43) Date of publication of application: **10.04.2019**
(21) Application number: 18202413.3
(22) Date of filing: 04.05.2009
(51) Int. Cl.: C12Q 1/6883

(54) **DETECTING GENETIC PREDISPOSITION TO OSTEOARTHRITIS ASSOCIATED CONDITIONS**

(30) Priority: 02.05.2008 US 4999208 P; 01.12.2008 US 11874408 P
(62) Divisional of application: 15193948.5
(71) Applicant: Orig3n, Inc., Boston, Massachusetts 02210 (US)
(72) Inventor: BUKOWSKI, Jack F., Marlborough, MA 01752-3334 (US); AZIZ, Nazneen, Lexington, MA 02142 (US); WANG, Hwa-Ying, Fremont, CA 94539 (US); HUTTNER, Kenneth, Chestnut Hill, MA 02467 (US); ABRAMSON, Steven B., Rye, NY 10580 (US); ATTUR, Mukundan, Woodside, NY 11377 (US)
(74) Representative: EIP

(57) **Abstract**

This application relates to methods and kits for detecting predisposition to increased risk for osteoarthritis associated conditions.

## Description

### RELATED APPLICATIONS

This application claims the benefit of provisional applications USSN 61/049,992, filed May 2, 2008, and USSN 61/118,744, filed December 1, 2008, the contents which are each herein incorporated by reference in their entirety.

### FIELD OF THE INVENTION

This invention relates to a method and kits for detecting a predisposition to, determining risk of, and guiding therapy for: incident osteoarthritis, osteoarthritis progression, osteoarthritis severity, -associated physical function decline, and disability.

### BACKGROUND

Osteoarthritis (OA) is a chronic joint disorder characterized by degeneration of joint cartilage and the adjacent bone. OA is generally considered a degenerative disease of aging, and the incidence rises with age. The etiology of osteoarthritis is multifactorial involving both mechanical and biochemical factors. Osteoarthritis commonly affects the hands, feet, spine, and large extraspinal, weight-bearing joints, such as the hips and knees. The joints predominantly involved are weight bearing and include the knees, hips, cervical and lumbosacral spine, and feet. Other commonly affected joints include the distal interphalangeal (DIP) and proximal interphalangeal (PIP) joints of the hands. Primary osteoarthritis generally refers to osteoarthritis of no known cause. Secondary osteoarthritis generally refers to osteoarthritis resulting from some external or internal injury or disease (obesity, repeated trauma or surgery to the joint structures, abnormal joints at birth (congenital abnormalities), gout, diabetes and other hormone disorders).

The structural progression of OA is currently assessed on plain radiographic views by measuring the joint space width (JSW) and/or joint space narrowing (JSN) over a period of time. (Altman et al.: Osteoarthritis Cartilage 1996, 4:217-243.) OA progression is associated with accelerated cartilage degradation leading to joint space narrowing, painful joint disruption, and functional compromise. OA disease progression is characterized by a proinflammatory gene expression pattern in cartilage and in joint synovium, with a reactive increase in bone density in the subchondral bone.

Osteoarthritis is the most common adult joint disease, affecting 5-20% of world's population and increasing in frequency and severity in all aging populations. The estimated U.S. prevalence is 15-60 million patients; 300-1200 million worldwide. OA involvement of the hand, knee, hip, and spine is common, with total knee replacements numbering over 300,000/yr in the U.S. and 700,000 additional worldwide. These numbers are expected to increase 525% by 2030. Further, Total hip replacements number over 150,000/yr in the U.S. alone. OA may involve a single joint or multiple joints in the same individual, with current therapy focused on pain relief as there is no FDA-approved therapy that arrests or reverses the joint deterioration.

Given the anticipated increase in osteoarthritis prevalence, there is a need to identify risk factors for incident osteoarthritis, osteoarthritis progression, osteoarthritis-associated physical function decline, and disability. Several studies have implicated factors, including genetic factors, aging, joint deformity and injury, obesity, and hormonal deficiencies in the pathogenesis of osteoarthritis. To optimize the management of OA, it is important to increase our knowledge regarding the predictors of progression of OA. Such prognostic factors may be used to identify high-risk groups for the development (or onset) of OA and/or high-risk groups for the severe disease progression of OA. Such patient information will be clinically useful for the medical management of OA patients. For example, if an individual with OA is known to be at increased risk for severe disease progression, the physician may initiate early treatment with disease modifying agents when they become available. Such prognostic information may also be clinically useful to guide decisions on the timing of joint replacement surgery. Knowledge about prognostic factors and an individual's predisposition for the onset and severe progression of the disease is also relevant for clinical research, such as for evaluating and developing therapeutic interventions including disease-modifying therapies. For example, since generally a small percentage of OA patients exhibit radiographic evidence of disease progression within a one to three year period, clinical trials of new therapeutic agents are challenging. Since a substantial portion of the experimental subjects will show no disease progression during the study, large numbers of subjects are traditionally needed to differentiate actives from placebo. In addition, because many treated subjects may have no measurable progression, and therefore no measurable drug benefit, the perceived value of an efficacious drug may be much lower than its actual value for patients who have progressive OA.

Large amounts of data provide support for a central role of interleukin-1 (TL-1) in the pathogenesis of OA including animal susceptibility models, models of IL-1-targeted therapy, and genetic association studies. ((Loughlin et al., Arthritis Rheum 2002;46(6):1519-27; Meulenbelt et al., Arthritis Rheum 2004;50(4):1179-86; Moos et al., Arthritis Rheum 2000;43(11):2417-22; Stern et al., Osteoarthritis Cartilage 2003;11(6):394-402; Smith et al., Genes Immun 2004;5(6):451-60; and Moxley et al., Osteoarthritis Cartilage 2007;15(10):1106-12.). For example, evidence from the literature suggests that genetic predisposition is an important determinant of pathology in patients with hand OA (Moxley et al.: Osteoarthritis Cartilage 2007;15(10):1106-12). Though a substantial literature exists on the role of IL-1 and to a lesser extent TNF-alpha (Botha-Scheepers et al., Ann Rheum Dis 2007.) on the pathogenesis of OA, less work has been done on genetic associations of these inflammatory mediators. Such associations are necessary to develop therapies that are appropriately targeted to subpopulations of OA patients whose disease is most likely to be responsive to IL-1 and TNF inhibitors. Further, there continues to be a need for a reliable marker to predict which osteoarthritis patients will experience severe disease progression.

### Genotype Screening

Traditional methods for the screening of heritable diseases have depended on either the identification of abnormal gene products (e.g., sickle cell anemia) or an abnormal phenotype (e.g., mental retardation). These methods are of limited utility for heritable diseases with late onset and no easily identifiable phenotypes such as, for example, vascular disease. With the development of simple and inexpensive genetic screening methodology, it is now possible to identify polymorphisms that indicate a propensity to develop disease, even when the disease is of polygenic origin. The number of diseases that can be screened by molecular biological methods continues to grow with increased understanding of the genetic basis of multifactorial disorders.

Genetic screening (also called genotyping or molecular screening), can be broadly defined as testing to determine if a patient has mutations (alleles or polymorphisms) that either cause a disease state or are "linked" to the mutation causing a disease state. Linkage refers to the phenomenon that DNA sequences which are close together in the genome have a tendency to be inherited together. Two sequences may be linked because of some selective advantage of co-inheritance. More typically, however, two polymorphic sequences are co-inherited because of the relative infrequency with which meiotic recombination events occur within the region between the two polymorphisms. The co-inherited polymorphic alleles are said to be in linkage disequilibrium with one another because, in a given human population, they tend to either both occur together or else not occur at all in any particular member of the population. Indeed, where multiple polymorphisms in a given chromosomal region are found to be in linkage disequilibrium with one another, they define a quasi-stable genetic "haplotype." In contrast, recombination events occurring between two polymorphic loci cause them to become separated onto distinct homologous chromosomes. If meiotic recombination between two physically linked polymorphisms occurs frequently enough, the two polymorphisms will appear to segregate independently and are said to be in linkage equilibrium.

While the frequency of meiotic recombination between two markers is generally proportional to the physical distance between them on the chromosome, the occurrence of "hot spots" as well as regions of repressed chromosomal recombination can result in discrepancies between the physical and recombinational distance between two markers. Thus, in certain chromosomal regions, multiple polymorphic loci spanning a broad chromosomal domain may be in linkage disequilibrium with one another, and thereby define a broad-spanning genetic haplotype. Furthermore, where a disease-causing mutation is found within or in linkage with this haplotype, one or more polymorphic alleles of the haplotype can be used as a diagnostic or prognostic indicator of the likelihood of developing the disease. This association between otherwise benign polymorphisms and a disease-causing polymorphism occurs if the disease mutation arose in the recent past, so that sufficient time has not elapsed for equilibrium to be achieved through recombination events. Therefore identification of a human haplotype which spans or is linked to a disease-causing mutational change, serves as a predictive measure of an individual's likelihood of having inherited that disease-causing mutation. Importantly, such prognostic or diagnostic procedures can be utilized without necessitating the identification and isolation of the actual disease-causing lesion. This is significant because the precise determination of the molecular defect involved in a disease process can be difficult and laborious, especially in the case of multifactorial diseases such as inflammatory disorders.

Indeed, the statistical correlation between an inflammatory disorder and an IL-1 polymorphism does not necessarily indicate that the polymorphism directly causes the disorder. Rather the correlated polymorphism may be a benign allelic variant which is linked to (i.e. in linkage disequilibrium with) a disorder-causing mutation which has occurred in the recent human evolutionary past, so that sufficient time has not elapsed for equilibrium to be achieved through recombination events in the intervening chromosomal segment. Thus, for the purposes of diagnostic and prognostic assays for a particular disease, detection of a polymorphic allele associated with that disease can be utilized without consideration of whether the polymorphism is directly involved in the etiology of the disease. Furthermore, where a given benign polymorphic locus is in linkage disequilibrium with an apparent disease-causing polymorphic locus, still other polymorphic loci which are in linkage disequilibrium with the benign polymorphic locus are also likely to be in linkage disequilibrium with the disease-causing polymorphic locus. Thus these other polymorphic loci will also be prognostic or diagnostic of the likelihood of having inherited the disease-causing polymorphic locus. Indeed, a broad-spanning human haplotype (describing the typical pattern of co-inheritance of alleles of a set of linked polymorphic markers) can be targeted for diagnostic purposes once an association has been drawn between a particular disease or condition and a corresponding human haplotype. Thus, the determination of an individual's likelihood for developing a particular disease of condition can be made by characterizing one or more disease-associated polymorphic alleles (or even one or more disease-associated haplotypes) without necessarily determining or characterizing the causative genetic variation.

### Genetics of the IL-1 Gene Cluster

The IL-1 gene cluster is on the long arm of chromosome 2 (2q13) and contains at least nine IL1 genes, including the well-described genes for IL-1 α (IL-1A), IL-1β (IL-1B), and the IL-1 receptor antagonist (IL-1RN), within a region of 430 Kb (Nicklin, et al. (1994) Genomics, 19: 3824; Dunn 2001; Sims 2001; Nicklin 2002). The agonist molecules, IL-1α and IL-1β, have potent pro-inflammatory activity and are at the head of many inflammatory cascades. Their actions, often via the induction of other cytokines such as IL-6 and IL-8, lead to activation and recruitment of leukocytes into damaged tissue, local production of vasoactive agents, and fever response in the brain and hepatic acute phase response. All three IL-1 molecules bind to type I and to type II IL-1 receptors, but only the type I receptor transduces a signal to the interior of the cell. In contrast, the type II receptor is shed from the cell membrane and acts as a decoy receptor. The receptor antagonist and the type II receptor, therefore, are both anti-inflammatory in their actions.

Inappropriate production of IL-1 plays a central role in the pathology of many autoimmune and inflammatory diseases, including rheumatoid arthritis, inflammatory bowel disorder, psoriasis, and the like. In addition, there are stable inter-individual differences in the rates of production of IL-1, and some of this variation may be accounted for by genetic differences at IL-1 gene loci. Thus, the IL-1 genes are reasonable candidates for determining part of the genetic susceptibility to inflammatory diseases, most of which have a multifactorial etiology with a polygenic component.

Certain alleles from the IL-1 gene cluster are known to be associated with particular disease states. For example, IL-1RN (VNTR) allele 2 (U.S. Pat. No. 5,698,399) and IL-1RN (VNTR) allele 1 (Keen R W et al., (1998) Bone 23:367-371) have been reported to be associated with OA. Further IL-1RN (VNTR) allele 2 has been reported to be associated with nephropathy in diabetes mellitus (Blakemore, et al. (1 996) Hum. Genet 97(3): 369-74), alopecia areata (Cork, et al., (1995) J. Invest. Dermatol. 104(5 Supp.): 15S-16S; Cork et al. (1996) Dermatol Clin 14: 671-8), Graves disease (Blakemore, et al. (1995) J. Clin. Endocrinol. 80(1): 111-5), systemic lupus erythematosus (Blakemore, et al. (1994) Arthritis Rheum. 37: 1380-85), lichen sclerosis (Clay, et al. (1994) Hum. Genet. 94: 407-10), and ulcerative colitis (Mansfield, et al. (1994) Gastoenterol. 106(3): 63742)).

In addition, the IL-1A allele 2 from marker -889 and IL-1B (TaqI) allele 2 from marker +3954 have been found to be associated with periodontal disease (U.S. Pat. No. 5,686,246; Kornman and diGiovine (1998) Ann Periodont 3: 327-38; Hart and Kornman (1997) Periodontol 2000 14: 202-15; Newman (1997) Compend Contin Educ Dent 18: 8814; Kornman et al. (1997) J. Clin Periodontol 24: 72-77). The IL-1A allele 2 from marker -889 has also been found to be associated with juvenile chronic arthritis, particularly chronic iridocyclitis (McDowell, et al. (1995) Arthritis Rheum. 38: 221-28). The IL-1B (TaqI) allele 2 from marker +3954 of IL-1B has also been found to be associated with psoriasis and insulin dependent diabetes in DR3/4 patients (di Giovine, et al. (1995) Cytokine 7: 606; Pociot, et al. (1992) Eur J. Clin. Invest. 22: 396-402). Additionally, the IL-1RN (VNTR) allele 1 has been found to be associated with diabetic retinopathy (see U.S. Ser. No. 09/037472, and PCT/GB97/02790). Furthermore allele 2 of IL-1RN (VNTR) has been found to be associated with ulcerative colitis in Caucasian populations from North America and Europe (Mansfield, J. et al., (1994) Gastroenterology 106: 637-42). Interestingly, this association is particularly strong within populations of ethnically related Ashkenazi Jews (PCT WO97/25445).

The description herein of disadvantages and problems associated with known compositions, and methods is in no way intended to limit the scope of the embodiments described in this document to their exclusion. Indeed, certain embodiments may include one or more known compositions, compounds, or methods without suffering from the so-noted disadvantages or problems.

Throughout this description, including the foregoing description of related art, any and all publicly available documents described herein, including any and all U. S. patents, are specifically incorporated by reference herein in their entirety. The foregoing description of related art is not intended in any way as an admission that any of the documents described therein, including pending United States patent applications, are prior art to the present invention.

### SUMMARY OF THE INVENTION

The present invention provides novel methods and kits for determining whether a subject is predisposed to developing OA and/or OA-related conditions. In one aspect, the present invention provides for novel methods and kits for determining whether a subject having OA is predisposed to increased risk of severe disease progression of OA. In another aspect, the present invention provides for novel methods and kits for determining whether a subject is predisposed to decreased risk of severe disease progression of OA.

In yet another aspect, the present invention provides for novel methods and kits for determining whether a subject is predisposed to increased risk of OA-associated physical function decline. In yet another aspect, the present invention provides for novel methods and kits for determining whether a subject is predisposed to decreased risk of osteoarthritis-associated physical function decline. OA-associated physical function decline may be determined using any method known in the art, such as by measuring joint space width (JSW) and/or joint space narrowing (JSN). Accordingly, another aspect of the invention provides for novel methods and kits for determining whether a subject is predisposed to increased risk of joint space narrowing. In yet another aspect, the present invention provides for novel methods and kits for determining whether a subject is predisposed to decreased risk of joint space narrowing.

According to some embodiments, methods are provided for detecting predisposition for increased risk of severe disease progression and/or joint space narrowing of osteoarthritis in a subject comprising detecting any one, any two, or all three of the following: a) genotype G/G at IL1RN rs9005 G>A; b) genotype T/T at IL1RN rs419598 T>C; and/or c) genotype T/C or C/C at IL1RN rs315952 T>C; wherein the presence of any one, any two, or all three of these genotypes indicates that the subject is predisposed to severe disease progression of osteoarthritis and the absence of all three of these genotypes indicates that the subject is not predisposed to severe disease progression of osteoarthritis.

According to some embodiments, methods are provided for detecting predisposition for decreased risk of severe disease progression and joint space narrowing of osteoarthritis in a subject comprising detecting in the subject either one or two copies of the following haplotype: IL1RN rs9005 G>A (A), IL1RN rs419598 T>C (C), IL-RN rs 315952 (T).

According to some embodiments, methods are provided for selecting osteoarthritis subjects for inclusion in or exclusion from clinical trials based on the likelihood of their disease progression and joint space narrowing of osteoarthritis comprising typing the subject's nucleic acid at one or more of the polymorphic loci selected from the group consisting of IL1RN rs9005 G>A, IL1RN rs419598 T>C, and IL1RN rs315952 T>C, wherein the subject's genotype with respect said loci provides information about the subject's risk for severe disease progression of osteoarthritis, and allows the selection of study subjects that are suitable for the criteria of the clinical trial.

According to some embodiments, methods are provided for selecting an appropriate therapeutic regimen for a subject suffering from osteoarthritis comprising typing the subject's nucleic acid at one or more of the polymorphic loci selected from the group consisting of IL1RN rs9005 G>A, IL1RN rs419598 T>C, and IL1RN rs315952 T>C, wherein the subject's genotype with respect said loci provides information about the subject's risk for severe disease progression and joint space narrowing of osteoarthritis, and allows the selection of a therapeutic regimen or lifestyle recommendation that is suitable to the subject's susceptibility to severe disease progression of osteoarthritis.

According to some embodiments, methods are provided for treating or slowing the disease progression and joint space narrowing of osteoarthritis in a subject comprising: a) detecting in the subject any one, any two, or all three of the following: genotype G/G at IL1RN rs9005 G>A; genotype T/T at IL1RN rs419598 T>C; and/or genotype T/C or C/C at IL1RN rs315952 T>C; and b) administering to said subject a therapeutic that compensates for the disease progression and joint space narrowing of osteoarthritis.

According to some embodiments, methods are provided for medical management of osteoarthritis by age stratification comprising detecting in a subject any one, any two, or all three of the following: a) genotype G/G at IL1RN rs9005 G>A; b) genotype T/T at IL1RN rs419598 T>C; and/or c) genotype T/C or C/C at IL1RN rs315952 T>C; wherein the presence of any one, any two, or all three of these genotype indicates that the subject is predisposed to severe disease progression and joint space narrowing of osteoarthritis and the absence of the genotype indicates that the subject is not predisposed to severe disease progression or joint space narrowing of osteoarthritis, and providing recommendations for the medical management of osteoarthritis based on the subject predicted needs at certain ages.

According to some embodiments, the kits of the present invention may include a means for determining whether a subject carries at least one allele comprising an OA associate allele or haplotype. The kit may also contain a sample collection means. The kit may also contain a control sample either positive or negative or a standard and/or an algorithmic device for assessing the results and additional reagents and components.

Other embodiments and advantages of the invention are set forth in the following detailed description and claims.

### DETAILED DESCRIPTION OF THE PREFERRED EMBODIMENTS

The present invention is based at least in part, on the identification of certain inflammatory alleles and haplotype patterns and the association (to a statistically significant extent) of these patterns with the development of OA related conditions such as disease progression of OA, and JSN progression. Therefore, detection of the alleles comprising OA associated alleles and haplotypes in a subject can indicate that the subject has or is predisposed to the development of a particular OA related condition. Osteoarthritis is also known by many other names: degenerative joint disease, hypertrophic arthritis, traumatic arthritis and osteoarthritis. (Rottensten K., Chronic Dis Can. 1996;17(3-4):92-107.)

Disease progression of OA can be defined in terms of degree of disability, radiographic worsening of OA, and/or the requirement for surgery (Dougados M., Arthritis Rheum. 2004 May;50(5): 1360-5). Disease progression in osteoarthritis is usually slow, and occurs over years or decades. The rate of progression is variable between individuals, and many patients with clinically diagnosed osteoarthritis may not suffer appreciable progression by either symptoms or radiographic changes over long periods. Severe radiographic progression is the most feared complication of OA, as it suggests irreversible joint destruction.

Thus, according to some embodiments, methods are provided for selecting an appropriate therapeutic regimen for a subject suffering from osteoarthritis comprising typing the subject's nucleic acid at one or more of the polymorphic loci selected from the group consisting of IL1RN rs9005 G>A, IL1RN rs419598 T>C, and IL1RN rs315952 T>C, wherein the subject's genotype with respect said loci provides information about the subject's risk for severe disease progression and joint space narrowing of osteoarthritis, and allows the selection of a therapeutic regimen or lifestyle recommendation that is suitable to the subject's susceptibility to severe disease progression of osteoarthritis. Treatment regimens or preventative measures may be selected to aggressively treat or prevent the progression of OA in subjects predisposed to increased risk of OA conditions. The methods of the present invention thus permit individualized. For example, a subject having any one, any two, or all three of the following: genotype G/G at IL1RN rs9005 G>A; genotype T/T at IL1RN rs419598 T>C; and/or genotype T/C or C/C at IL1RN rs315952 T>C has a predisposition for an increased risk for the progression and would benefit from an aggressive treatment/prevention regime that would compensate for that subject's relative increased rate for disease progression and joint space narrowing of osteoarthritis.

According to some embodiments, methods arc provided for selecting an appropriate therapeutic regimen for a subject suffering from osteoarthritis comprising typing the subject's nucleic acid at one or more of the polymorphic loci selected from the group consisting of ILIA (+4845) rs17561 (G>T), IL1B(+3954) rs1143634 (C>T), IL1B(-511) rs16944(C>T), IL1B(-3737) rs4848306 (C>T), TNF-α(-308) rs1800629(G>A), and IL-1RN rs315952 (T>C), wherein the subject's genotype with respect said loci provides information about the subject's risk for developing generalized OA, and allows the selection of a therapeutic regimen or lifestyle recommendation that is suitable to the subject's susceptibility to severe disease progression of osteoarthritis. Treatment regimens or preventative measures may be selected to aggressively treat or prevent the progression of OA in subjects predisposed to increased risk of OA conditions. The methods of the present invention thus permit individualized therapy. Valuable medical resources may thus be focused on those subjects most likely to experience severe disease progression of OA.

Knowledge of the particular alleles associated with a susceptibility to developing osteoarthritis, alone or in conjunction with information on other contributing factors of OA allows a customization of the prevention or treatment in accordance with the individual's genetic profile, the goal of "pharmacogenomics". Thus, comparison of an individual's genetic profile to the population profile for osteoarthritis, permits the selection of drugs or other therapeutic regimens that are expected to be safe and efficacious for a particular patient or patient population (i.e., a group of patients having the same genetic alteration). Medical resources may be focused early on those patients who are at risk for progression and severe disease.

Any treatment or preventive regimen requires a level of commitment in behalf of the physician and subject. The methods of the present invention help to ensure that the required level of attention is given to those predisposed to increased risk for OA associated conditions. Such subjects may choose to aggressively use disease-modifying osteoarthritis drugs (DMOADs), also referred to as structure-modifying osteoarthritis drugs (SMOADs). DMOAD or OA therapeutics refers to any agent or therapeutic regimen (including pharmaceuticals, nutraceuticals and surgical means) that prevents or postpones the development of or alleviates the symptoms of osteoarthritis in the subject. The therapeutic can be a polypeptide, peptidomimetic, nucleic acid or other inorganic or organic molecule, preferably a "small molecule" including vitamins, minerals and other nutrients. DMOAD include, but are not limited to, glucosamine, chondroitin sulfate, doxycycline, risedronate, diacerein, and IA hyaluronan. The methods of the present invention will help physicians, patients, and insurance companies decide who needs these modalities.

Another issue is joint replacement surgery. The replacement joint only lasts 15 years, so difficult second surgeries are often necessary in younger patients. The methods of the current invention may be used to manage the OA treatment based on the likelihood of radiographic progression. The methods of the current invention may be used to assess the likelihood that a subject will require a first or second joint replacement surgery.

Age is often a consideration when deciding the timing of surgery. Age also influences that progression of disease. For the purposes of medical management of OA, age may be divided into strata with treatment options or medical outcomes assigned to each strata. In this manner, the progression of the disease may be managed across the subject's lifetime. Four age strata may be defined as < 40 years, 40-55 years, 56-70 years, and >70 years.

Several grading scales arc available to those of skill in the art to correlate radiographic grade of osteoarthritis with the actual degree of articular cartilage degeneration within any particular joint. These include, but are not limited to, Kellgren-Lawrence, Ahlback, and Brandt grading scales. See Kellgren J, Lawrence J. Radiologic assessment of osteoarthritis. Ann Rheum Dis 1957;16 : 494-501; Ahlback S. Osteoarthritis of the knee: a radiographic investigation. Acta Radiol Diagn (Stockh) 1968; [suppl 227]: 7-72; Brandt K, Fife R, Braunstein E, Katz B. Radiographic grading of the severity of knee osteoarthritis: relation of the Kellgren and Lawrence grade to a grade based on joint space narrowing and correlation with arthroscopic evidence of articular cartilage degeneration. Arthritis Rheum 1989; 32:1584 -1591. The Kellgren-Lawrence is the preferred method for assessing the presence and severity of osteoarthritis. A brief explanation of the Kellgren-Lawrence Radiographic Grading Scale and Brandt Radiographic Grading Scale are provided below. A person of skill in the art would appreciate the differences in the criteria and/or methodology of these and other grading systems known in the art, it is also appreciated that these grading systems are equivalent for assessing the presence and severity of osteoarthritis.

### Kellgren-Lawrence Radiographic Grading Scale of Osteoarthritis

| Grade of Osteoarthritis | | |
|---|---|---|
| | | |

| | Description | |
|---|---|---|
| | | |
| | 0 - none | No radiographic findings of osteoarthritis |
| | 1 - Doubtful | Minute osteophytes of doubtful clinical significance |
| | 2 - Minimal | Definite osteophytes with unimpaired joint space |
| | 3 - Moderate | Definite osteophytes with moderate joint space narrowing |
| | 4 - Severe | Definite osteophytes with severe joint space narrowing and subchondral sclerosis |
| | | |

### Brandt Radiographic Grading Scale of Osteoarthritis

| Grade of Osteoarthritis | | |
|---|---|---|
| | | |

| | Description | |
|---|---|---|
| | | |
| | 0 - none | No radiographic findings of osteoarthritis |
| | 1 - Doubtful | < 25% joint space narrowing with secondary features |
| | 2 - Minimal | 50-75% joint space narrowing without secondary features |
| | 3 - Moderate | 50-75% joint space narrowing with secondary features |
| | 4 - Severe | > 75% joint space narrowing with secondary features |
| | | |

### Measurement of OA Progression and JSW Progression.

Preservation of the integrity of the articular cartilage is generally considered an important aspect of OA to be measured in assessing the efficacy of any treatment and as an important measure of assessing the disease progression of OA. Interbone distance in the plain radiograph is the best available surrogate measure for articular cartilage thickness. Loss of joint space within the knee has been equated with loss of articular cartilage. It is therefore common for physicians to quantify this deterioration by measuring the amount of space between the different components of the joint. A narrowing of the joint space indicates worsening osteoarthritis. Joint space narrowing (JSN) is often utilized as an outcome measure for standardizing the radioanatomic position of the joint in serial radiologic examinations.

Joint space narrowing or thinning of articular cartilage associated with OA may be measured using any method known in the art. Magnetic resonance imaging is a preferred method to monitor joint structure. Double-contrast arthrography radiographic joint space width (JSW) is a preferred methods. Continuous measures of JSW are preferred. Alternatively, minimum JSW may be used (*i.e.,* measured medial compartment JSW at the narrowest point). OA progression may be measured by comparing the frequency with which subjects exhibit loss of JSW. This frequency or other measure for the rate of JSN may be use to measure OA progression.

### Clinical Trials

There are several studies reporting the clinical trials of purported disease-modifying OA drugs (DMOADs). The methods of the present invention will enable an investigator to select study subjects that are at an increased risk or decreased risk (depending on the study's objectives) of disease progression of OA. According to one embodiment, methods are provided for selecting osteoarthritis subjects for inclusion in clinical trials based on their predisposition for disease progression and increased rate of joint space narrowing of osteoarthritis. Subjects having a genotype indicating that the subject is predisposed for increased risks of disease progression and/or joint space narrowing of osteoarthritis may be selected into a study researching the efficacy of certain DMOADs. The methods of the present invention may be used to select study subjects for clinical trials researching the efficacy and safety of therapies for OA, disease progression of OA, and/or rate of JSN.

In addition, the ability to target populations expected to show the highest clinical benefit, based on genetic profile can enable: 1) the repositioning of already marketed drugs; 2) the rescue of drug candidates whose clinical development has been discontinued as a result of safety or efficacy limitations, which are patient subgroup-specific; and 3) an accelerated and less costly development for candidate therapeutics and more optimal drug labeling (e.g. since measuring the effect of various doses of an agent on the causative mutation is useful for optimizing effective dose).

### Detection of Alleles

Haplotype patterns can be identified by detecting any of the component alleles using any of a variety of available techniques, including: 1) performing a hybridization reaction between a nucleic acid sample and a probe that is capable of hybridizing to the allele; 2) sequencing at least a portion of the allele; or 3) determining the electrophoretic mobility of the allele or fragments thereof (e.g., fragments generated by endonuclease digestion). The allele can optionally be subjected to an amplification step prior to performance of the detection step. Preferred amplification methods are selected from the group consisting of: the polymerase chain reaction (PCR), the ligase chain reaction (LCR), strand displacement amplification (SDA), cloning, and variations of the above (e.g. RT-PCR and allele specific amplification). Oligonucleotides necessary for amplification may be selected, for example, from within the IL-1 gene loci, either flanking the marker of interest (as required for PCR amplification) or directly overlapping the marker (as in ASO hybridization). In a particularly preferred embodiment, the sample is hybridized with a set of primers, which hybridize 5' and 3' in a sense or antisense sequence to the vascular disease associated allele, and is subjected to a PCR amplification.

An allele may also be detected indirectly, e.g. by analyzing the protein product encoded by the DNA. For example, where the marker in question results in the translation of a mutant protein, the protein can be detected by any of a variety of protein detection methods. Such methods include immunodetection and biochemical tests, such as size fractionation, where the protein has a change in apparent molecular weight either through truncation, elongation, altered folding or altered post-translational modifications.

A general guideline for designing primers for amplification of unique human chromosomal genomic sequences is that they possess a melting temperature of at least about 50 °C., wherein an approximate melting temperature can be estimated using the formula Tₘₑₗₜ=[2X(# of A or T)+4X(# of G or C)].

Many methods are available for detecting specific alleles at human polymorphic loci. The preferred method for detecting a specific polymorphic allele will depend, in part, upon the molecular nature of the polymorphism. For example, the various allelic forms of the polymorphic locus may differ by a single base-pair of the DNA. Such single nucleotide polymorphisms (or SNPs) are major contributors to genetic variation, comprising some 80% of all known polymorphisms, and their density in the human genome is estimated to be on average 1 per 1,000 base pairs. SNPs are most frequently biallelic-occurring in only two different forms (although up to four different forms of an SNP, corresponding to the four different nucleotide bases occurring in DNA, are theoretically possible). Nevertheless, SNPs are mutationally more stable than other polymorphisms, making them suitable for association studies in which linkage disequilibrium between markers and an unknown variant is used to map disease-causing mutations. In addition, because SNPs typically have only two alleles, they can be genotyped by a simple plus/minus assay rather than a length measurement, making them more amenable to automation.

A variety of methods are available for detecting the presence of a particular single nucleotide polymorphic allele in an individual. Advancements in this field have provided accurate, easy, and inexpensive large-scale SNP genotyping. Most recently, for example, several new techniques have been described including dynamic allele-specific hybridization (DASH), microplate array diagonal gel electrophoresis (MADGE), pyrosequencing, oligonucleotide-specific ligation, the TaqMan system as well as various DNA "chip" technologies such as the Affymetrix SNP chips. These methods require amplification of the target genetic region, typically by PCR. Still other newly developed methods, based on the generation of small signal molecules by invasive cleavage followed by mass spectrometry or immobilized padlock probes and rolling-circle amplification, might eventually eliminate the need for PCR. Several of the methods known in the art for detecting specific single nucleotide polymorphisms are summarized below. The method of the present invention is understood to include all available methods.

Several methods have been developed to facilitate analysis of single nucleotide polymorphisms. In one embodiment, the single base polymorphism can be detected by using a specialized exonuclease-resistant nucleotide, as disclosed, e.g., in Mundy, C. R. (U.S. Pat. No.4,656,127). According to the method, a primer complementary to the allelic sequence immediately 3' to the polymorphic site is permitted to hybridize to a target molecule obtained from a particular animal or human. If the polymorphic site on the target molecule contains a nucleotide that is complementary to the particular exonuclease-resistant nucleotide derivative present, then that derivative will be incorporated onto the end of the hybridized primer. Such incorporation renders the primer resistant to exonuclease, and thereby permits its detection. Since the identity of the exonuclease-resistant derivative of the sample is known, a finding that the primer has become resistant to exonucleases reveals that the nucleotide present in the polymorphic site of the target molecule was complementary to that of the nucleotide derivative used in the reaction. This method has the advantage that it does not require the determination of large amounts of extraneous sequence data.

In another embodiment of the invention, a solution-based method is used for determining the identity of the nucleotide of a polymorphic site. Cohen, D. et al. (French Patent 2,650,840; PCT Appln. No. WO91/02087). As in the Mundy method of U.S. Pat. No. 4,656,127, a primer is employed that is complementary to allelic sequences immediately 3' to a polymorphic site. The method determines the identity of the nucleotide of that site using labeled dideoxynucleotide derivatives, which, if complementary to the nucleotide of the polymorphic site will become incorporated onto the terminus of the primer.

An alternative method, known as Genetic Bit Analysis or GBA.TM. is described by Goelet, P. et al. (PCT Appln. No. 92/15712). The method of Goelet, P. et al. uses mixtures of labeled terminators and a primer that is complementary to the sequence 3' to a polymorphic site. The labeled terminator that is incorporated is thus determined by, and complementary to, the nucleotide present in the polymorphic site of the target molecule being evaluated. In contrast to the method of Cohen et al. (French Patent 2,650,840; PCT Appln. No. WO91/02087) the method of Goelet, P. et al. is preferably a heterogeneous phase assay, in which the primer or the target molecule is immobilized to a solid phase.

Recently, several primer-guided nucleotide incorporation procedures for assaying polymorphic sites in DNA have been described (Komher, J. S. et al., Nucl. Acids. Res. 17:7779-7784 (1989); Sokolov, B. P., Nucl. Acids Res. 18:3671 (1990); Syvanen, A.-C., et al., Genomics 8:684-692 (1990); Kuppuswamy, M. N. et al., Proc. Natl. Acad. Sci. (U.S.A.) 88:1143-1147 (1991); Prezant, T. R. et al., Hum. Mutat. 1:159-164 (1992); Ugozzoli, L. et al., GATA 9:107-112 (1992); Nyren, P. et al., Anal. Biochem. 208:171-175 (1993)). These methods differ from GBA.TM. in that they all rely on the incorporation of labeled deoxynucleotides to discriminate between bases at a polymorphic site. In such a format, since the signal is proportional to the number of deoxynucleotides incorporated, polymorphisms that occur in runs of the same nucleotide can result in signals that are proportional to the length of the run (Syvanen, A.-C., et al., Amer. J. Hum. Genet. 52:46-59 (1993)).

For mutations that produce premature termination of protein translation, the protein truncation test (PTT) offers an efficient diagnostic approach (Roest, et. al., (1993) Hum. Mol. Genet. 2:1719-2 1; van der Luijt, et. al., (1994) Genomics 20:1-4). For PTT, RNA is initially isolated from available tissue and reverse-transcribed, and the segment of interest is amplified by PCR. The products of reverse transcription PCR are then used as a template for nested PCR amplification with a primer that contains an RNA polymerase promoter and a sequence for initiating eukaryotic translation. After amplification of the region of interest, the unique motifs incorporated into the primer permit sequential in vitro transcription and translation of the PCR products. Upon sodium dodecyl sulfate-polyacrylamide gel electrophoresis of translation products, the appearance of truncated polypeptides signals the presence of a mutation that causes premature termination of translation. In a variation of this technique, DNA (as opposed to RNA) is used as a PCR template when the target region of interest is derived from a single exon.

Any cell type or tissue may be utilized to obtain nucleic acid samples for use in the diagnostics described herein. In a preferred embodiment, the DNA sample is obtained from a bodily fluid, e.g., blood, obtained by known techniques (e.g. venipuncture) or saliva. Alternatively, nucleic acid tests can be performed on dry samples (e.g. hair or skin). When using RNA or protein, the cells or tissues that may be utilized must express an IL-1 gene.

Diagnostic procedures may also be performed in situ directly upon tissue sections (fixed and/or frozen) of patient tissue obtained from biopsies or resections, such that no nucleic acid purification is necessary. Nucleic acid reagents may be used as probes and/or primers for such in situ procedures (see, for example, Nuovo, G. J., 1992, PCR in situ hybridization: protocols and applications, Raven Press, NY).

In addition to methods which focus primarily on the detection of one nucleic acid sequence, profiles may also be assessed in such detection schemes. Fingerprint profiles may be generated, for example, by utilizing a differential display procedure, Northern analysis and/or RT-PCR.

A preferred detection method is allele specific hybridization using probes overlapping a region of at least one allele of an IL-1 proinflammatory haplotype and having about 5, 10, 20, 25, or 30 nucleotides around the mutation or polymorphic region. In a preferred embodiment of the invention, several probes capable of hybridizing specifically to other allelic variants involved in a restenosis are attached to a solid phase support, e.g., a "chip" (which can hold up to about 250,000 oligonucleotides). Oligonucleotides can be bound to a solid support by a variety of processes, including lithography. Mutation detection analysis using these chips comprising oligonucleotides, also termed "DNA probe arrays" is described e.g., in Cronin et al. (1996) Human Mutation 7:244. In one embodiment, a chip comprises all the allelic variants of at least one polymorphic region of a gene. The solid phase support is then contacted with a test nucleic acid and hybridization to the specific probes is detected. Accordingly, the identity of numerous allelic variants of one or more genes can be identified in a simple hybridization experiment.

These techniques may also comprise the step of amplifying the nucleic acid before analysis. Amplification techniques are known to those of skill in the art and include, but are not limited to cloning, polymerase chain reaction (PCR), polymerase chain reaction of specific alleles (ASA), ligase chain reaction (LCR), nested polymerase chain reaction, self sustained sequence replication (Guatelli, J. C. et al., 1990, Proc. Natl. Acad. Sci. USA 87:1874-1878), transcriptional amplification system (Kwoh, D. Y. et al., 1989, Proc. Natl. Acad. Sci. USA 86:1173-1177), and Q- Beta Replicase (Lizardi, P. M. ct al., 1988, Bio/Technology 6:1197).

Amplification products may be assayed in a variety of ways, including size analysis, restriction digestion followed by size analysis, detecting specific tagged oligonucleotide primers in the reaction products, allele-specific oligonucleotide (ASO) hybridization, allele specific 5' exonuclease detection, sequencing, hybridization, and the like.

PCR based detection means can include multiplex amplification of a plurality of markers simultaneously. For example, it is well known in the art to select PCR primers to generate PCR products that do not overlap in size and can be analyzed simultaneously. Alternatively, it is possible to amplify different markers with primers that are differentially labeled and thus can each be differentially detected. Of course, hybridization based detection means allow the differential detection of multiple PCR products in a sample. Other techniques are known in the art to allow multiplex analyses of a plurality of markers.

In a merely illustrative embodiment, the method includes the steps of (i) collecting a sample of cells from a patient, (ii) isolating nucleic acid (e.g., genomic, mRNA or both) from the cells of the sample, (iii) contacting the nucleic acid sample with one or more primers which specifically hybridize 5' and 3' to at least one allele of an IL-1 proinflammatory haplotype under conditions such that hybridization and amplification of the allele occurs, and (iv) detecting the amplification product. These detection schemes are especially useful for the detection of nucleic acid molecules if such molecules are present in very low numbers.

In a preferred embodiment of the subject assay, the allele of an IL-1 proinflammatory haplotype is identified by alterations in restriction enzyme cleavage patterns. For example, sample and control DNA is isolated, amplified (optionally), digested with one or more restriction endonucleases, and fragment length sizes are determined by gel electrophoresis.

In yet another embodiment, any of a variety- of sequencing reactions known in the art can be used to directly sequence the allele. Exemplary sequencing reactions include those based on techniques developed by Maxim and Gilbert ((1977) Proc. Natl Acad Sci USA 74:560) or Sanger (Sanger et al (1977) Proc. Nat. Acad. Sci USA 74:5463). It is also contemplated that any of a variety of automated sequencing procedures may be utilized when performing the subject assays (see, for example Biotechniques (1995) 19:448), including sequencing by mass spectrometry (see, for example PCT publication WO 94/16101; Cohen et al. (1996) Adv Chromatogr 36:127-162; and Griffin et al. (1993) Appl Biochem Biotechnol 38:147-159). It will be evident to one of skill in the art that, for certain embodiments, the occurrence of only one, two or three of the nucleic acid bases need be determined in the sequencing reaction. For instance, A-track or the like, e.g., where only one nucleic acid is detected, can be carried out.

In a further embodiment, protection from cleavage agents (such as a nuclease, hydroxylamine or osmium tetroxide and with piperidine) can be used to detect mismatched bases in RNA/RNA or RNA/DNA or DNA/DNA heteroduplexes (Myers, et al. (1985) Science 230:1242). In general, the art technique of "mismatch cleavage" starts by providing heteroduplexes formed by hybridizing (labeled) RNA or DNA containing the wild-type allele with the sample. The double-stranded duplexes are treated with an agent which cleaves single-stranded regions of the duplex such as which will exist due to base pair mismatches between the control and sample strands. For instance, RNA/DNA duplexes can be treated with RNase and DNA/DNA hybrids treated with S1 nuclease to enzymatically digest the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al (1988) Proc. Natl Acad Sci USA 85:4397; and Saleeba et al (1992) Methods Enzymol. 217:286-295. In a preferred embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes). For example, the mutY enzyme of E. coli cleaves A at G/A mismatches and the thymidine DNA glycosylase from HeLa cells cleaves T at G/T mismatches (Hsu et al. (1994) Carcinogenesis 15:1657-1662). According to an exemplary embodiment, a probe based on an allele of an IL-1 locus haplotype is hybridized to a CDNA or other DNA product from a test cell(s). The duplex is treated with a DNA mismatch repair enzyme, and the cleavage products, if any, can be detected from electrophoresis protocols or the like. See, for example, U.S. Pat. No. 5,459,039.

In other embodiments, alterations in electrophoretic mobility will be used to identify an IL-1 locus allele. For example, single strand conformation polymorphism (SSCP) may be used to detect differences in electrophoretic mobility between mutant and wild type nucleic acids (Orita et al. (1989) Proc Natl. Acad. Sci USA 86:2766, see also Cotton (1993) Mutat Res 285:125-144; and Hayashi (1992) Genet Anal Tech Appl 9:73-79). Single-stranded DNA fragments of sample and control IL-1 locus alleles are denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) Trends Genet 7:5).

In yet another embodiment, the movement of alleles in polyacrylamide gels containing a gradient of denaturant is assayed using denaturing gradient gel electrophoresis (DGGE) (Myers et al. (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing agent gradient to identify differences in the mobility of control and sample DNA (Rosenbaum and Reissner (1987) Biophys Chem 265:12753).

Examples of other techniques for detecting alleles include, but are not limited to, selective oligonucleotide hybridization, selective amplification, or selective primer extension. For example, oligonucleotide primers may be prepared in which the known mutation or nucleotide difference (e.g., in allelic variants) is placed centrally and then hybridized to target DNA under conditions which permit hybridization only if a perfect match is found (Saiki et al. (1986) Nature 324:163); Saiki et al (1989) Proc. Natl Acad. Sci USA 86:6230). Such allele specific oligonucleotide hybridization techniques may be used to test one mutation or polymorphic region per reaction when oligonucleotides are hybridized to PCR amplified target DNA or a number of different mutations or polymorphic regions when the oligonucleotides are attached to the hybridizing membrane and hybridized with labelled target DNA.

Alternatively, allele specific amplification technology which depends on selective PCR amplification may be used in conjunction with the instant invention. Oligonucleotides used as primers for specific amplification may carry the mutation or polymorphic region of interest in the center of the molecule (so that amplification depends on differential hybridization) (Gibbs et al (1989) Nucleic Acids Res. 17:2437-2448) or at the extreme 3' end of one primer where, under appropriate conditions, mismatch can prevent, or reduce polymerase extension (Prossner (1993) Tibtech 1 1:238. In addition it may be desirable to introduce a novel restriction site in the region of the mutation to create cleavage-based detection (Gasparini et al (1992) Mol. Cell Probes 6:1). It is anticipated that in certain embodiments amplification may also be performed using Taq ligase for amplification (Barany (1991) Proc. Natl. Acad. Sci USA 88:189). In such cases, ligation will occur only if there is a perfect match at the 3' end of the 5' sequence making it possible to detect the presence of a known mutation at a specific site by looking for the presence or absence of amplification.

In another embodiment, identification of the allelic variant is carried out using an oligonucleotide ligation assay (OLA), as described, e.g., in U.S. Pat. No. 4,998,617 and in Landegren, U. et al. ((1988) Science 241:1077-1080). The OLA protocol uses two oligonucleotides which are designed to be capable of hybridizing to abutting sequences of a single strand of a target. One of the oligonucleotides is linked to a separation marker, e.g,. biotinylated, and the other is detectably labeled. If the precise complementary sequence is found in a target molecule, the oligonucleotides will hybridize such that their termini abut, and create a ligation substrate. Ligation then permits the labeled oligonucleotide to be recovered using avidin, or another biotin ligand. Nickerson, D. A. et al. have described a nucleic acid detection assay that combines attributes of PCR and OLA (Nickerson, D. A. et al. (1990) Proc. Natl. Acad. Sci. USA 87:8923-27). In this method, PCR is used to achieve the exponential amplification of target DNA, which is then detected using OLA.

Several techniques based on this OLA method have been developed and can be used to detect alleles of an IL-1 locus haplotype. For example, U.S. Pat. No. 5,593,826 discloses an OLA using an oligonucleotide having 3'-amino group and a 5'-phosphorylated oligonucleotide to form a conjugate having a phosphoramidate linkage. In another variation of OLA described in Tobe et al. ((1996) Nucleic Acids Res 24: 3728), OLA combined with PCR permits typing of two alleles in a single microtiter well. By marking each of the allele-specific primers with a unique hapten, i.e. digoxigenin and fluorescein, each OLA reaction can be detected by using hapten specific antibodies that are labeled with different enzyme reporters, alkaline phosphatase or horseradish peroxidase. This system permits the detection of the two alleles using a high throughput format that leads to the production of two different colors.

Another embodiment of the invention is directed to kits for detecting a predisposition for developing a restenosis. This kit may contain one or more oligonucleotides, including 5' and 3' oligonucleotides that hybridize 5' and 3' to at least one allele of an IL-1 locus haplotype. PCR amplification oligonucleotides should hybridize between 25 and 2500 base pairs apart, preferably between about 100 and about 500 bases apart, in order to produce a PCR product of convenient size for subsequent analysis.

Particularly preferred primers for use in the diagnostic method of the invention include SEQ ID NO: 1-25.

| SNP | SEQ ID NO: | Purpose | Sequence |
|---|---|---|---|
| IL1RN rs9005 G>A | SEQ ID NO: 1 | PCR | TGAGCAAATGTGGCTCCTGG GGGTTCT |
| | SEQ ID NO: 2 | PCR | CCCAAAGCCTGTCAAGGCCA AGGACAT |
| | SEQ ID NO: 3 | SBE | GATGGCTGTGCCTCTGCCTGT CTCCCCCACC |
| | | | |
| IL1RN rs419598 T>C | SEQ ID NO: 4 | PCR | ACAAGTTCTGGGGGACACAG |
| | SEQ ID NO: 5 | PCR | AGGCCATGCTGCTGCAGACA |
| | SEQ ID NO: 6 | SBE | GACCTTCTATCTGAGGAACA ACCAACTAGTTGC |
| | | | |
| IL1RN rs315952 T>C | SEQ ID NO: 7 | PCR | GCCTCAGCTCTCACCTGCCCA TCTTTTG |
| | SEQ ID NO: 8 | PCR | AGGCAGCATGGAGGCTGGTC AGTTGAA |
| | SEQ ID NO: 9 | SBE | GACAAGCGCTTCGCCTTCATC CGCTCAGACAG |

| | | | |
|---|---|---|---|
| PCR = Polymerase Chain Reaction SBE = Single Base Extension genotyping | | | |

The design of additional oligonucleotides for use in the amplification and detection of IL-1 polymorphic alleles by the method of the invention is facilitated by the availability of both updated sequence information from human chromosome 2q13--which contains the human IL-1 locus, and updated human polymorphism information available for this locus. For example, the DNA sequence for the IL-1A, IL-1B and IL-1RN can be found at the National Center for Biotechnology Information website (http://www.ncbi.nlm.nih.gov/) using GenBank Accession No. X03833, No. X04500 and No. X64532 respectively. Suitable primers for the detection of a human polymorphism in these genes can be readily designed using this sequence information and standard techniques known in the art for the design and optimization of primers sequences. Optimal design of such primer sequences can be achieved, for example, by the use of commercially available primer selection programs such as Primer 2.1, Primer 3 or GeneFisher (See also, Nicklin M. H. J., Weith A. Duff G. W., "A Physical Map of the Region Encompassing the Human Interleukin-la, interleukin-1β, and Interleukin-1 Receptor Antagonist Genes" Genomics 19: 382 (1995); Nothwang H. G., et al. "Molecular Cloning of the Interleukin-1 gene Cluster: Construction of an Integrated YAC/PAC Contig and a partial transcriptional Map in the Region of Chromosome 2q13" Genomics 41: 370 (1997); Clark, et al. (1986) Nucl. Acids. Res., 14:7897-7914 [published erratum appears in Nucleic Acids Res., 15:868 (1987) and the Genome Database (GDB) project).

In another aspect, the invention features kits for performing the above-described assays. According to some embodiments, the kits of the present invention may include a means for determining whether a subject carries at least one allele comprising an OA associate allele or haplotype. The kit may also contain a nucleic acid sample collection means. The kit may also contain a control sample either positive or negative or a standard and/or an algorithmic device for assessing the results and additional reagents and components including: DNA amplification reagents, DNA polymerase, nucleic acid amplification reagents, restrictive enzymes, buffers, a nucleic acid sampling device, DNA purification device, deoxynucleotides, oligonucleotides (e.g. probes and primers) etc.

For use in a kit, oligonucleotides may be any of a variety of natural and/or synthetic compositions such as synthetic oligonucleotides, restriction fragments, cDNAs, synthetic peptide nucleic acids (PNAs), and the like. The assay kit and method may also employ labeled oligonucleotides to allow ease of identification in the assays. Examples of labels which may be employed include radio-labels, enzymes, fluorescent compounds, streptavidin, avidin, biotin, magnetic moieties, metal binding moieties, antigen or antibody moieties, and the like.

As described above, the control may be a positive or negative control. Further, the control sample may contain the positive (or negative) products of the allele detection technique employed. For example, where the allele detection technique is PCR amplification, followed by size fractionation, the control sample may comprise DNA fragments of the appropriate size. Likewise, where the allele detection technique involves detection of a mutated protein, the control sample may comprise a sample of mutated protein. However, it is preferred that the control sample comprises the material to be tested. For example, the controls may be a sample of genomic DNA or a cloned portion of the IL-1 gene cluster. Preferably, however, the control sample is a highly purified sample of genomic DNA where the sample to be tested is genomic DNA.

The oligonucleotides present in said kit may be used for amplification of the region of interest or for direct allele specific oligonucleotide (ASO) hybridization to the markers in question. Thus, the oligonucleotides may either flank the marker of interest (as required for PCR amplification) or directly overlap the marker (as in ASO hybridization).

Information obtained using the assays and kits described herein (alone or in conjunction with information on another genetic defect or environmental factor, which contributes to osteoarthritis) is useful for determining whether a non-symptomatic subject has or is likely to develop the particular disease or condition. In addition, the information can allow a more customized approach to preventing the onset or progression of the disease or condition. For example, this information can enable a clinician to more effectively prescribe a therapy that will address the molecular basis of the disease or condition.

The kit may, optionally, also include DNA sampling means. DNA sampling means arc well known to one of skill in the art and can include, but not be limited to substrates, such as filter papers, the AmpliCard.TM. (University of Sheffield, Sheffield, England S10 2JF; Tarlow, J W, et al., J. of Invest. Dermatol. 103:387-389 (1994)) and the like; DNA purification reagents such as Nucleon.TM. kits, lysis buffers, proteinase solutions and the like; PCR reagents, such as 10X reaction buffers, thernostable polymerase, dNTPs, and the like; and allele detection means such as the Hinfl restriction enzyme, allele specific oligonucleotides, degenerate oligonucleotide primers for nested PCR from dried blood.

### Definitions

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In the case of conflict, the present specification, including definitions, will control. In addition, the materials, methods, and examples arc illustrative only not intended to be limiting. Other features and advantages of the invention will be apparent from the following detailed description and claims.

For the purposes of promoting an understanding of the embodiments described herein, reference will be made to preferred embodiments and specific language will be used to describe the same. The terminology used herein is for the purpose of describing particular embodiments only, and is not intended to limit the scope of the present invention. As used throughout this disclosure, the singular forms "a," "an," and "the" include plural reference unless the context clearly dictates otherwise. Thus, for example, a reference to "a composition" includes a plurality of such compositions, as well as a single composition, and a reference to "a therapeutic agent" is a reference to one or more therapeutic and/or pharmaceutical agents and equivalents thereof known to those skilled in the art, and so forth.

The term "allele" refers to the different sequence variants found at different polymorphic regions. For example, IL-1RN (VNTR) has at least five different alleles. The sequence variants may be single or multiple base changes, including without limitation insertions, deletions, or substitutions, or may be a variable number of sequence repeats.

The term "allelic pattern" refers to the identity of an allele or alleles at one or more polymorphic regions. For example, an allelic pattern may consist of a single allele at a polymorphic site, as for IL-1RN (VNTR) allele 1, which is an allelic pattern having at least one copy of IL-1 RN allele 1 at the VNTR of the IL-1RN gene loci. Alternatively, an allelic pattern may consist of either a homozygous or heterozygous state at a single polymorphic site. For example, IL-1-RN (VNTR) allele 2,2 is an allelic pattern in which there are two copies of the second allele at the VNTR marker of IL-1RN that corresponds to the homozygous IL-RN (VNTR) allele 2 state. Alternatively, an allelic pattern may consist of the identity of alleles at more than one polymorphic site.

"Biological activity" or "bioactivity" or "activity" or "biological function", which are used interchangeably, for the purposes herein means an effector or antigenic function that is directly or indirectly performed by an IL-1 polypeptide (whether in its native or denatured conformation), or by any subsequence thereof. Biological activities include binding to a target peptide, e.g., an IL-1 receptor. An IL-1 bioactivity can be modulated by directly affecting an IL-1 polypeptide. Alternatively, an IL-1 bioactivity can be modulated by modulating the level of an IL-1 polypeptide, such as by modulating expression of an IL-1 gene.

As used herein the term "bioactive fragment of an IL-1 polypeptide" refers to a fragment of a full-length IL-1 polypeptide, wherein the fragment specifically mimics or antagonizes the activity of a wild-type IL-1 polypeptide. The bioactive fragment preferably is a fragment capable of interacting with an interleukin receptor.

The terms "control" or "control sample" refer to any sample appropriate to the detection technique employed. The control sample may contain the products of the allele detection technique employed or the material to be tested. Further, the controls may be positive or negative controls. By way of example, where the allele detection technique is PCR amplification, followed by size fractionation, the control sample may comprise DNA fragments of an appropriate size. Likewise, where the allele detection technique involves detection of a mutated protein, the control sample may comprise a sample of a mutant protein. However, it is preferred that the control sample comprises the material to be tested. For example, the controls may be a sample of genomic DNA or a cloned portion of the IL-1 gene cluster. However, where the sample to be tested is genomic DNA, the control sample is preferably a highly purified sample of genomic DNA.

The phrases "disruption of the gene" and "targeted disruption" or any similar phrase refers to the site specific interruption of a native DNA sequence so as to prevent expression of that gene in the cell as compared to the wild-type copy of the gene. The interruption may be caused by deletions, insertions or modifications to the gene, or any combination thereof.

The term "haplotype" as used herein is intended to refer to a set of alleles that are inherited together as a group (are in linkage disequilibrium) at statistically significant levels (P_{corr} <0.05). As used herein, the phrase "an IL-1 haplotype" refers to a haplotype in the IL-1 loci. An IL-1 inflammatory or proinflammatory haplotype refers to a haplotype that is indicative of increased agonist and/or decreased antagonist activities.

The terms "IL-1 gene cluster" and "IL-1 loci" as used herein include all the nucleic acid at or near the 2q13 region of chromosome 2, including at least the IL-1A, IL-1B and IL-1RN genes and any other linked sequences. (Nicklin et al., Genomics 19: 382-84, 1994). The terms "IL-1A", "IL-1B", and "IL-1RN" as used herein refer to the genes coding for IL-1 alpha, IL-1 beta, and IL-1 receptor antagonist, respectively. The gene accession number for IL-1A, IL-1B, and IL-1RN are X03833, X04500, and X64532, respectively.

"IL-1 functional mutation" refers to a mutation within the IL-1 gene cluster that results in an altered phenotype (i.e. effects the function of an IL-1 gene or protein). Examples include: IL-1A(+4845) allele 2, IL-1B (-3737) allele 2, IL-1B (+6912) allele 2, IL-1B(-31) allele 2, and IL-1RN (+2018) allele 2.

"IL-1 X (Z) allele Y" refers to a particular allelic form, designated Y, occurring at an IL-1 locus polymorphic site in gene X, wherein X is IL-1 A, B, or RN and positioned at or near nucleotide Z, wherein nucleotide Z is numbered relative to the major transcriptional start site, which is nucleotide +1, of the particular IL-1 gene X. As further used herein, the term "IL-1 X allele (Z)" refers to all alleles of an IL-1 polymorphic site in gene X positioned at or near nucleotide Z. For example, the term "IL-1RN (+2018) allele" refers to alternative forms of the IL-1RN gene at marker +2018. "IL-1RN (+2018) allele 2" refers to a form of the IL-1RN gene which contains a cytosine (C) at position +2018 of the sense strand. Clay et al., Hum. Genet. 97:723-26, 1996. "IL-1RN (+2018) allele 1" refers to a form of the IL-1 RN gene which contains a thymine (T) at position +2018 of the plus strand. When a subject has two identical IL-1RN alleles, the subject is said to be homozygous, or to have the homozygous state. When a subject has two different IL-1RN alleles, the subject is said to be heterozygous, or to have the heterozygous state. The term "IL-1RN (+2018) allele 2,2" refers to the homozygous IL-1RN (+2018) allele 2 state. Conversely, the term "IL-1RN (+2018) allele 1,1" refers to the homozygous IL-1RN (+2018) allele 1 state. The term "IL-1RN (+2018) allele 1,2" refers to the heterozygous allele 1 and 2 state.

Alternatively, an allele is named by the nucleotide at the polymorphic site. For example, "IL-1RN (+2018) allele T" refers to a form of the IL-1 RN gene which contains a thymine (T) at position +2018 of the plus strand.

"IL-1 related" as used herein is meant to include all genes related to the human IL-1 locus genes on human chromosome 2 (2q 12-14). These include IL-1 genes of the human IL-1 gene cluster located at chromosome 2 (2q 13-14) which include: the IL-1 A gene which encodes interleukin-la, the IL-1B gene which encodes interleukin-1β, and the IL-1RN (or IL-1ra) gene which encodes the interleukin-1 receptor antagonist. Furthermore these IL-1 related genes include the type I and type II human IL-1 receptor genes located on human chromosome 2 (2q12) and their mouse homologs located on mouse chromosome 1 at position 19.5 cM. Interleukin-la, interleukin-1β, and interleukin-1RN are related in so much as they all bind to IL-1 type I receptors, however only interleukin-la and interleukin-1β are agonist ligands which activate IL-1 type I receptors, while interleukin-1RN is a naturally occurring antagonist ligand. Where the term "IL-1" is used in reference to a gene product or polypeptide, it is meant to refer to all gene products encoded by the interleukin-1 locus on human chromosome 2 (2q 12-14) and their corresponding homologs from other species or functional variants thereof. The term IL-1 thus includes secreted polypeptides which promote an inflammatory response, such as IL-1α and IL-1β, as well as a secreted polypeptide which antagonize inflammatory responses, such as IL-1 receptor antagonist and the IL-1 type II (decoy) receptor.

An "IL-1 receptor" or "IL-1 R" refers to various cell membrane bound protein receptors capable of binding to and/or transducing a signal from an IL-1 locus-encoded ligand. The term applies to any of the proteins which are capable of binding interleukin-1 (IL-1) molecules and, in their native configuration as mammalian plasma membrane proteins, presumably play a role in transducing the signal provided by IL-1 to a cell. As used herein, the term includes analogs of native proteins with IL-1 -binding or signal transducing activity. Examples include the human and murine IL-1 receptors described in U.S. Pat. No. 4,968,607. The term "IL-1 nucleic acid" refers to a nucleic acid encoding an IL-1 protein.

An "IL-1 polypeptide" and "IL-1 protein" are intended to encompass polypeptides comprising the amino acid sequence encoded by the IL-1 genomic DNA sequences identified by GenBank accession numbers X03833, X04500, and X64532, or fragments thereof, and homologs thereof and include agonist and antagonist polypeptides.

"Increased risk" refers to a statistically higher frequency of occurrence of the disease or condition in an individual carrying a particular polymorphic allele in comparison to the frequency of occurrence of the disease or condition in a member of a population that does not carry the particular polymorphic allele.

The term "interact" as used herein is meant to include detectable relationships or associations (e.g. biochemical interactions) between molecules, such as interactions between protein-protein, protein-nucleic acid, nucleic acid-nucleic acid and protein-small molecule or nucleic acid-small molecule in nature.

The term "isolated" as used herein with respect to nucleic acids, such as DNA or RNA, refers to molecules separated from other DNAs, or RNAs, respectively, that are present in the natural source of the macromolecule. For example, an isolated nucleic acid encoding one of the subject IL-1 polypeptides preferably includes no more than 10 kilobases (kb) of nucleic acid sequence which naturally immediately flanks the IL-1 gene in genomic DNA, more preferably no more than 5 kb of such naturally occurring flanking sequences, and most preferably less than 1.5 kb of such naturally occurring flanking sequence. The term isolated as used herein also refers to a nucleic acid or peptide that is substantially free of cellular material, viral material, or culture medium when produced by recombinant DNA techniques, or chemical precursors or other chemicals when chemically synthesized. Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides.

"Linkage disequilibrium" refers to co-inheritance of two alleles at frequencies greater than would be expected from the separate frequencies of occurrence of each allele in a given control population. The expected frequency of occurrence of two alleles that are inherited independently is the frequency of the first allele multiplied by the frequency of the second allele. Alleles that co-occur at expected frequencies are said to be in "linkage disequilibrium". The cause of linkage disequilibrium is often unclear. It can be due to selection for certain allele combinations or to recent admixture of genetically heterogeneous populations. In addition, in the case of markers that are very tightly linked to a disease gene, an association of an allele (or group of linked alleles) with the disease gene is expected if the disease mutation occurred in the recent past, so that sufficient time has not elapsed for equilibrium to be achieved through recombination events in the specific chromosomal region. When referring to allelic patterns that are comprised of more than one allele, a first allelic pattern is in linkage disequilibrium with a second allelic pattern if all the alleles that comprise the first allelic pattern are in linkage disequilibrium with at least one of the alleles of the second allelic pattern. An example of linkage disequilibrium is that which occurs between the alleles at the IL-1RN (+2018) and IL-1RN (VNTR) polymorphic sites. The two alleles at IL-1RN (+2018) are 100% in linkage disequilibrium with the two most frequent alleles of IL-1RN (VNTR), which are allele 1 and allele 2.

The term "marker" refers to a sequence in the genome that is known to vary among individuals. For example, the IL-1RN gene has a marker that consists of a variable number of tandem repeats (VNTR).

A "mutated gene" or "mutation" or "functional mutation" refers to an allelic form of a gene, which is capable of altering the phenotype of a subject having the mutated gene relative to a subject which does not have the mutated gene. The altered phenotype caused by a mutation can be corrected or compensated for by certain agents. If a subject must be homozygous for this mutation to have an altered phenotype, the mutation is said to be recessive. If one copy of the mutated gene is sufficient to alter the phenotype of the subject, the mutation is said to be dominant. If a subject has one copy of the mutated gene and has a phenotype that is intermediate between that of a subject who is homozygous for the wild type gene and that of a subject homozygous for the mutated gene, the mutation is said to be co-dominant.

A "non-human animal" of the invention includes mammals such as rodents, non-human primates, sheep, dogs, cows, goats, etc. amphibians, such a s members of the Xenopus genus, and transgenic avians (e.g. chickens, birds, etc.). The term "chimeric animal" is used herein to refer to animals in which the recombinant gene is found, or in which the recombinant gene is expressed in some but not all cells of the animal. The term "tissue-specific chimeric animal" indicates that one of the recombinant IL-1 genes is present and/or expressed or disrupted in some tissues but not others. The term "non-human mammal" refers to any member of the class Mammalia, except for humans.

As used herein, the term "nucleic acid" refers to polynucleotides or oligonucleotides such as deoxyribonucleic acid (DNA), and, where appropriate, ribonucleic acid (RNA). The term should also be understood to include, as equivalents, analogs of either RNA or DNA made from nucleotide analogs (e.g. peptide nucleic acids) and as applicable to the embodiment being described, single (sense or antisense) and double-stranded polynucleotides.

The term "polymorphism" refers to the coexistence of more than one form of a gene or portion (e.g., allelic variant) thereof. A portion of a gene of which there are at least two different forms, i.e., two different nucleotide sequences, is referred to as a "polymorphic region of a gene". A specific genetic sequence at a polymorphic region of a gene is an allele. A polymorphic region can be a single nucleotide, the identity of which differs in different alleles. A polymorphic region can also be several nucleotides long.

The term "propensity to disease," also "predisposition" or "susceptibility" to disease or any similar phrase, means that certain alleles are hereby discovered to be associated with or predictive of a subject's incidence of developing a particular disease (e.g. a vascular disease). The alleles arc thus over-represented in frequency in individuals with disease as compared to healthy individuals. Thus, these alleles can be used to predict disease even in pre-symptomatic or pre-diseased individuals.

"Small molecule" as used herein, is meant to refer to a composition, which has a molecular weight of less than about 5 kD and most preferably less than about 4 kD. Small molecules can be nucleic acids, peptides, peptidomimetics, carbohydrates, lipids or other organic or inorganic molecules.

As used herein, the term "specifically hybridizes" or "specifically detects" refers to the ability of a nucleic acid molecule to hybridize to at least approximately 6 consecutive nucleotides of a sample nucleic acid.

"Transcriptional regulatory sequence" is a generic term used throughout the specification to refer to DNA sequences, such as initiation signals, enhancers, and promoters, which induce or control transcription of protein coding sequences with which they are operably linked.

As used herein, the term "transgene" means a nucleic acid sequence (encoding, e.g., one of the IL-1 polypeptides, or an antisense transcript thereto) which has been introduced into a cell. A transgene could be partly or entirely heterologous, i.e., foreign, to the transgenic animal or cell into which it is introduced, or, is homologous to an endogenous gene of the transgenic animal or cell into which it is introduced, but which is designed to be inserted, or is inserted, into the animal's genome in such a way as to alter the genome of the cell into which it is inserted (e.g., it is inserted at a location which differs from that of the natural gene or its insertion results in a knockout). A transgene can also be present in a cell in the form of an episome. A transgene can include one or more transcriptional regulatory sequences and any other nucleic acid, such as introns, that may be necessary for optimal expression of a selected nucleic acid.

The term "treating" as used herein is intended to encompass curing as well as ameliorating at least one symptom of a condition or disease.

The term "vector" refers to a nucleic acid molecule, which is capable of transporting another nucleic acid to which it has been linked. One type of preferred vector is an episome, i.e., a nucleic acid capable of extra-chromosomal replication. Preferred vectors are those capable of autonomous replication and/or expression of nucleic acids to which they are linked. Vectors capable of directing the expression of genes to which they arc operatively linked are referred to herein as "expression vectors". In general, expression vectors of utility in recombinant DNA techniques are often in the form of "plasmids" which refer generally to circular double stranded DNA loops which, in their vector form are not bound to the chromosome. In the present specification, "plasmid" and "vector" are used interchangeably as the plasmid is the most commonly used form of vector. However, the invention is intended to include such other forms of expression vectors which serve equivalent functions and which become known in the art subsequently hereto.

The term "wild-type allele" refers to an allele of a gene which, when present in two copies in a subject results in a wild-type phenotype. There can be several different wild-type alleles of a specific gene, since certain nucleotide changes in a gene may not affect the phenotype of a subject having two copies of the gene with the nucleotide changes.

The following examples arc illustrative, but not limiting, of the methods and compositions of the present invention. Other suitable modifications and adaptations of the variety of conditions and parameters normally encountered in therapy and that are obvious to those skilled in the art are within the spirit and scope of the embodiments.

### EXAMPLE 1

IL-1 RN Polymorphisms are Associated with Radiographic Severity in Osteoarthritis.

. Background/purpose: There continues to be a need for reliable markers to predict which osteoarthritis (OA) patients will experience disease progression. Previous studies have suggested that inflammation may be important in the pathogenesis and progression of OA. We performed a study in subjects with knee OA to investigate selected candidate gene polymorphisms for associations with radiographic severity.

Methods: Eighty OA patients with knee OA from NYUHJD, met inclusion criteria in this cross-sectional retrospective analysis. Caucasians of either sex, free of chronic disease, with a radiographic diagnosis of knee OA were genotyped for single nucleotide polymorphisms (SNPs). These subjects were divided into two groups: one having index knee Kellgren-Lawrence (KL) scores of one or two, and the other, KL scores of three or four. A subset) with available data (N=36) was separated by joint space width (JSW) either above or below the median. We performed statistical analysis using Chi square and Fisher's exact test, with logistic regression, adjusting for age, gender and BMI to evaluate associations of radiographic severity with individual SNPs and with haplotypes.

Results: After adjustment for age, gender, and BMI, individual IL1 receptor antagonist (IL1RN) SNPs were strongly associated with KL radiographic severity and joint space width (JSW; Table 1). Also, carriage of either one or two copies of a haplotype consisting of (ACT): IL1RN rs9005, IL1RN rs419598, IL1RN rs315952 (haplotype frequency in this OA study 32%) was associated with a substantially decreased risk of radiographic severity (OR 0.14; 95% CI 0.05-0.37), and with increased JSW (3.99± 1.76 mm vs 3.16±1.94mm; p= 0.0056) compared to the remaining 68% of the OA population.

Conclusion: IL1RN SNPs are associated with radiographic severity in OA, and may predict the potential for radiographic severity and progression. These genetic markers may be useful for the medical management of OA, and also as a tool to enrich for subjects who progress radiographically in clinical trials of DMOADs.

**Table 1. Association of SNPs with Radiographic Severity**

| | | | **KL score >2 (N=80)** | | **JSW<median (N=36)** | |
|---|---|---|---|---|---|---|
| | | | | **OR (95% CI)** | **p** | **OR (95% CI)** |
| **SNP** | **Genotype** | **Frequency** | **p** | | | |
| IL1RN rs9005 G>A | GG | 55% | *<0.0 002* | *6.51 (2.33-18.17)* | *0.007* | *14.5 (2.05-103.1)* |
| | AA/AG | 45% | | 1.0 | | 1.0 |
| IL1RN rs419598 T>C | TT | 65% | *0.14* | *2.05 (0.77-5.41)* | *0.016* | *28.7 (1.86-445.0)* |
| | CC/CT | 35% | | 1.0 | | 1.0 |
| IL1RN rs315952 T>C | CC/CT | 47% | *0.015* | *3.09 (1.22-1.79)* | *NS* | *NS* |
| | TT | 52% | | 1.0 | | |

While the invention has been described with reference to particularly preferred embodiments and examples, those skilled in the art recognize that various modifications may be made to the invention without departing from the spirit and scope thereof.

All of the above U.S. patents, U.S. patent application publications, U.S. patent applications, foreign patents, foreign patent applications and non-patent publications referred to in this specification and/or listed in the Application Data Sheet are incorporated herein by reference, in their entirety.

### Genotype definitions:

| | |
|---|---|
| IL1RN_rs315952 | T/T 1.1 |
| | T/C 1.2 |
| | C/C 2.2 |
| IL1RN_rs9005 | G/G 1.1 |
| | G/A 1.2 |
| | A/A 2.2 |
| IL1RN+(2018)_rs419598 | T/T 1.1 |
| | T/C 1.2 |
| | C/C 2.2 |

### IL-1RN haplotype pairs:

| Gene symbol (HGNC) | | |
|---|---|---|
| IL1RN | Chromosome | 2 |
| | Location | 2q14.2 |
| | Chr 2 pos | 113607883(+) |
| | dbSNP ID | rs9005 |
| | Sequence | ccacc**G**/**A**ggctg |
| | 5' primer (5' to 3') | |
| | 3'primer (5' to 3') | |
| | Allele 1 | G |
| | Allele 2 | A |
| | | |
| IL1RN | Chromosome | 2 |
| | Location | 2q14.2 |
| | Chr 2 pos | 113603678(+) |
| | dbSNP ID | rs419598 |
| | Sequence | gttgc**C**/**T**ggata |
| | 5' primer (5' to 3') | |
| | 3'primer (5' to 3') | |
| | Allele 1 | T |
| | Allele 2 | C |
| | | |
| IL1RN | Chromosome | 2 |
| | Location | 2q14.2 |
| | Chr 2 pos | 113606775(+) |
| | dbSNP ID | rs315952 |
| | Sequence | gacag**C**/**T**ggccc |
| | 5' primer (5' to 3') | |
| | 3'primer (5' to 3') | |
| | Allele 1 | T |
| | Allele 2 | C |

### Reference list

1. Loughlin J, Dowling B, Mustafa Z, Chapman K. Association of the interleukin-1 gene cluster on chromosome 2q13 with knee osteoarthritis. Arthritis Rheum 2002;46(6):1519-27.
2. Meulenbelt I, Seymour AB, Nieuwland M, Huizinga TW, van Duijn CM, Slagboom PE. Association of the interleukin-1 gene cluster with radiographic signs of osteoarthritis of the hip. Arthritis Rheum 2004;50(4):1179-86.
3. Moos V, Rudwaleit M, Herzog V, Hohlig K, Sieper J, Muller B. Association of genotypes affecting the expression of interleukin-1beta or interleukin-1 receptor antagonist with osteoarthritis. Arthritis Rheum 2000;43(11):2417-22.
4. Stern AG, de Carvalho MR, Buck GA, Adler RA, Rao TP, Disler D, et al. Association of erosive hand osteoarthritis with a single nucleotide polymorphism on the gene encoding interleukin-1 beta. Osteoarthritis Cartilage 2003;11(6):394-402.
5. Smith AJ, Keen LJ, Billingham MJ, Perry MJ, Elson CJ, Kirwan JR, et al. Extended haplotypes and linkage disequilibrium in the IL1R1-IL1A-IL1B-IL1RN gene cluster: association with knee osteoarthritis. Genes Immun 2004;5(6):451-60.
7. Moxley G, Han J, Stern AG, Riley BP. Potential influence of IL1B haplotype and IL1AIL1B-IL1RN extended haplotype on hand osteoarthritis risk. Osteoarthritis Cartilage 2007;15(10):1106-12.
8. Botha-Scheepers SA, Watt I, Slagboom E, de Craen AJ, Meulenbelt I, Rosendaal FR, et al. Innate production of Tumor Necrosis Factor-{alpha} and Interleukin-10 is associated with radiological progression of knee osteoarthritis. Ann Rheum Dis 2007.

### EMBODIMENTS

1. A method of detecting predisposition for increased risk of severe disease progression and joint space narrowing of osteoarthritis in a subject comprising detecting in the subject any one, any two, or all three of the following:
   a) genotype G/G at IL1RN rs9005 G>A;
   b) genotype T/T at IL1RN rs419598 T>C;
   c) genotype T/C or C/C at IL1RN rs315952 T>C;
   wherein the presence of any one, any two, or all three of these genotypes indicates that the subject is predisposed to the severe disease progression, joint space narrowing, and other biomarkers of disease severity, including, but not limited to, cytokines, chemokines, collagen fragments, and proteinases of osteoarthritis and the absence of all three of these genotypes indicates that the subject is not predisposed to such.
2. A method of detecting predisposition for decreased risk of severe disease progression and joint space narrowing of osteoarthritis in a subject comprising detecting in the subject:
   a) genotype A/A or G/A at IL1RN rs9005 G>A;
   b) genotype C/C or T/C at IL1RN rs419598 T>C;
   c) genotype T/T at IL1RN rs315952 T>C;
   wherein the presence of any one, any two, or all three of these genotypes indicates that the subject is protected from progression to severe disease, joint space narrowing, and other biomarkers of disease severity, including, but not limited to, cytokines, chemokines, collagen fragments, and proteinases, of osteoarthritis and the absence of all of these genotypes indicates that the subject is not protected from such.
3. A method of detecting predisposition for increased risk of severe disease progression, joint space narrowing, and other biomarkers of disease severity, including, but not limited to, cytokines, chemokines, collagen fragments, and proteinases of osteoarthritis in a subject comprising detecting in the subject no copies of the following haplotype (ACT): IL1RN rs9005 G>A, IL1RN rs419598 T>C, IL1RN rs315952 T>C whereby the presence of one or two copies of this haplotype predicts reduced risk of severe disease progression and associated conditions.
4. A method for selecting osteoarthritis subjects for inclusion in or exclusion from clinical trials based on the likelihood of their disease progression, joint space narrowing, and other biomarkers of disease severity, including, but not limited to, cytokines, chemokines, collagen fragments, and proteinases of osteoarthritis comprising typing the subject's nucleic acid at one or more of the polymorphic loci selected from the group consisting of IL1RN rs9005 G>A, IL1RN rs419598 T>C, and IL1RN rs315952 T>C, wherein the subject's genotype with respect to said loci provides information about the subject's risk for such, and allows the selection of study subjects that are suitable for the criteria of the clinical trial.
5. A method for selecting an appropriate therapeutic regimen, based on, but not limited to, therapeutic classes of disease-modifying osteoarthritis drugs (DMOADs) for a subject suffering from osteoarthritis comprising typing the subject's nucleic acid at one or more of the polymorphic loci selected from the group consisting of IL1RN rs9005 G>A, IL1RN rs419598 T>C, and IL1RN rs315952 T>C, wherein the subject's genotype with respect to said loci provides information about the subject's risk for severe disease progression, joint space narrowing, and other biomarkers of disease severity, including, but not limited to, cytokines, chemokines, collagen fragments, and proteinases, of osteoarthritis, and allows the selection of a therapeutic regimen or lifestyle recommendation that is suitable to the subject's susceptibility to such.
6. A method for treating or slowing the disease progression and joint space narrowing of osteoarthritis in a subject comprising:
   a) detecting in the subject any one, any two, or all three of the following: genotype G/G at IL1RN rs9005 G>A; genotype T/T at IL1RN rs419598 T>C; and/or genotype T/C or C/C at IL1RN rs315952 T>C;
   b) administering to said subject a therapeutic that compensates for the disease progression, joint space narrowing, and other biomarkers of disease severity, including, but not limited to, cytokines, chemokines, collagen fragments, and proteinases of osteoarthritis.
7. A method of according to any one of the preceding embodiment, wherein said subject is over about 60 years of age.
8. A method of according to any one of the preceding embodiment, wherein said subject is over about 40 years of age.
9. A method of according to any one of the preceding embodiment, wherein said subject is between about 40 and about 60 years of age.
10. A method of according to any one of the preceding embodiment, wherein said subject is between about 65 and about 90 years of age.
11. A method of according to any one of the preceding embodiment, wherein said detecting step is selected from the group consisting of: a) allele specific oligonucleotide hybridization; b) size analysis; c) sequencing; d) hybridization; e) 5' nuclease digestion; f) single-stranded conformation polymorphism; g) allele specific hybridization; h) primer specific extension; and i) oligonucleotide ligation assay.
12. A method of according to any one of the preceding embodiment, wherein prior to or in conjunction with detection, the nucleic acid sample is subject to an amplification step.
13. A kit for detecting predisposition for increased or decreased risk for severe disease progression of osteoarthritis and joint space narrowing in a subject, said kit comprising a primer oligonucleotide that hybridizes 5' or 3' to an allele selected from the group consisting of IL1RN rs9005 G>A, IL1RN rs419598 T>C, and IL1RN rs315952 T>C.
14. The kit of embodiment 14, further comprising a second primer oligonucleotide that hybridizes either 3' or 5' respectively to the allele, such that the allele can be amplified.
15. The kit of embodiment 14, wherein said primer hybridizes to a region in the range of between about 50 and about 1000 base pairs.
16. The kit of embodiment 14, further comprising a detection means.
17. The kit of embodiment 14, further comprising an amplification means.
18. The kit of embodiment 14, further comprising a control.
19. A method for medical management, and predicting rate of disease progression of osteoarthritis by age consideration and stratification, comprising
   detecting in a subject any one, any two, or all three of the following:
   a) genotype G/G at IL1RN rs9005 G>A;
   b) genotype T/T at IL1RN rs419598 T>C; and/or
   c) genotype C/T or C/C at IL1RN rs315952 T>C;
   wherein the presence of any one, any two, or all three of these genotype indicates that the subject is predisposed to severe disease progression, joint space narrowing, and other biomarkers of disease severity, including, but not limited to, cytokines, chemokines, collagen fragments, and proteinases, of osteoarthritis and the absence of the genotype indicates that the subject is not predisposed to such, and providing recommendations for the medical management of osteoarthritis based on the subject's predicted needs at certain ages.
20. A method for medical management, and prediction of rate of disease progression of osteoarthritis by age consideration and stratification, comprising detecting predisposition for decreased risk of severe disease progression, joint space narrowing, and other biomarkers of disease severity, including, but not limited to, cytokines, chemokines, collagen fragments, and proteinases of osteoarthritis in a subject comprising detecting in the subject one or two copies of the following haplotype (ACT): IL1RN rs9005 G>A, IL1RN rs419598 T>C, IL1RN rs315952 T>C and the absence of the haplotype indicates that the subject is not predisposed to such, and providing recommendations for the medical management of osteoarthritis based on the subject predicted needs at certain ages.
21. A method for medical management of osteoarthritis by age consideration comprising detecting predisposition for protection against severe disease progression, joint space narrowing, and other biomarkers of disease severity, including, but not limited to, cytokines, chemokines, collagen fragments, and proteinases, of osteoarthritis in a subject comprising detecting in the subject one or two copies of the following haplotype: (ACT): IL1RN rs9005 G>A, IL1RN rs419598 T>C, IL1RN rs315952 T>C and the absence of the haplotype indicates that the subject is not predisposed for such, and providing recommendations for the medical management of osteoarthritis based on the subject predicted needs at certain ages.

## Claims

1. A method of determining predisposition for osteoarthritis disease progression and joint space narrowing related to osteoarthritis in a subject comprising detecting in the subject the genotype at IL1RN rs315952 T>C;
wherein the presence of genotype *T*/*C* or *C*/*C* indicates that the subject is predisposed to disease progression and joint space narrowing; and
wherein the presence of IL1RN rs315952 genotype *T*/*T* indicates that the subject is protected from disease progression and joint space narrowing.

2. The method of claim 1, further comprising detecting in the subject the genotype at IL1RN rs419598 T>C and IL1RN rs9005 G>A;
wherein the presence of IL1RN rs419598 genotype *T*/*T,* IL1RN rs9005 genotype *G*/*G,* and IL1RN rs315952 genotype *T*/*C or C*/*C* indicates that the subject is predisposed to disease progression and joint space narrowing; and
wherein the presence of IL1RN rs419598 genotype *C*/*C* or *T*/*C,* IL1RN rs9005 genotype A/A or *G*/*A,* and IL1RN rs315952 genotype *T*/*T* indicates that the subject is protected from disease progression and joint space narrowing.

3. A method for selecting osteoarthritis subjects for inclusion in or exclusion from a clinical trial comprising determining predisposition for osteoarthritis disease progression and joint space narrowing related to osteoarthritis in a subject according to any one of the preceding claims and including or excluding said subject from the clinical trial according to the subject's risk and criteria of the clinical trial.

4. A method according to any one of the preceding claims, wherein said detecting step is selected from the group consisting of: a) allele specific oligonucleotide hybridization; b) size analysis; c) sequencing; d) hybridization; e) 5' nuclease digestion; f) single-stranded conformation polymorphism; g) allele specific hybridization; h) primer specific extension; and i) oligonucleotide ligation assay.

5. A method according to any one of the preceding claims, wherein prior to or in conjunction with said detecting step, the nucleic acid sample is subject to an amplification step.

6. The method of any one of claims 1 to 2, further comprising providing recommendations for the medical management of osteoarthritis based on the subject's predicted needs at certain ages.


SEQUENCE LISTING

<110>  Interleukin Genetics, Inc.

<120>  Detecting Genetic Predisposition to Osteoarthritis Associated
       Conditions

<130>  ILGN-038D01EP

<140>  Divisional of EP09740020.4
<141>  2009-05-04

<150>  61/049,992
<151>  2008-05-02

<150>  61/118,744
<151>  2008-12-01

<160>  9

<170>  PatentIn version 3.5

<210>  1
<211>  27
<212>  DNA
<213>  Artificial Sequence

<220>
<223>  SEQ ID NO: 1; primer sequence

<400>  1
tgagcaaatg tggctcctgg gggttct                                          27


<210>  2
<211>  27
<212>  DNA
<213>  Artificial Sequence

<220>
<223>  SEQ ID NO: 2; primer sequence

<400>  2
cccaaagcct gtcaaggcca aggacat                                          27



<210>  3
<211>  31
<212>  DNA
<213>  Artificial Sequence

<220>
<223>  SEQ ID NO: 3; primer sequence

<400>  3
gatggctgtg cctctgcctg tctcccccac c                                     31


<210>  4
<211>  20
<212>  DNA

<220>
<223>  SEQ ID NO: 4; primer sequence

<400>  4
acaagttctg ggggacacag                                                    20


<210>  5
<211>  20
<212>  DNA
<213>  Artificial Sequence

<220>
<223>  SEQ ID NO: 5; primer sequence

<400>  5
aggccatgct gctgcagaca                                                    20


<210>  6
<211>  33
<212>  DNA
<213>  Artificial Sequence

<220>
<223>  SEQ ID NO: 6; primer sequence

<400>  6
gaccttctat ctgaggaaca accaactagt tgc                                     33


<210>  7
<211>  28
<212>  DNA
<213>  Artificial Sequence

<220>
<223>  SEQ ID NO: 7; primer sequence

<400>  7
gcctcagctc tcacctgccc atcttttg                                           28


<210>  8
<211>  27
<212>  DNA
<213>  Artificial Sequence

<220>
<223>  SEQ ID NO: 8; primer sequence

<400>  8
aggcagcatg gaggctggtc agttgaa                                            27


<210>  9
<211>  32
<212>  DNA
<213>  Artificial Sequence

<220>

<400> 9
gacaagcgct tcgccttcat ccgctcagac ag 32